# EUROPEAN PATENT SPECIFICATION

(11) **EP 2 160 181 B1**
(45) Date of publication and mention of the grant of the patent: **17.08.2016**
(21) Application number: 08760339.5
(22) Date of filing: 02.06.2008
(51) Int. Cl.: A61K 8/34, A61K 8/41, A61K 8/891, A61K 8/892, A61K 8/06, A61Q 5/12

(54) **HAIR CARE COMPOSITIONS**
HAARPFLEGEZUSAMMENSETZUNGEN
COMPOSITIONS DE SOIN CAPILLAIRE

(30) Priority: 02.07.2007 EP 07111515
(43) Date of publication of application: 10.03.2010
(73) Proprietor: Unilever PLC, London, Greater London EC4Y 0DY (GB); Unilever N.V., 3013 AL Rotterdam (NL)
(72) Inventor: AVERY, Andrew, Richard, Bebington Wirral Merseyside CH63 3JW (GB)
(74) Representative: Chisem, Janet
(86) International application number: PCT/EP2008/056752
(87) International publication number: WO 2009/003775

(56) References cited:
- WO-A-91/09586
- WO-A-99/53889
- WO-A-2005/060923
- WO-A-2006/058755
- WO-A1-03/092637
- US-A- 5 162 378
- DATABASE GNPD [Online] MINTEL August 2005 'Crema Styler Anti-Frizz Hair Cream Reformulation' Database accession no. 10227264

## Description

### FIELD OF THE INVENTION

This invention relates to hair care compositions incorporating alkyl modified silicones.

### BACKGROUND AND PRIOR ART

Alkyl modified silicones have found application within a range of cosmetic products, particularly as moisturisers in skin care products and as spreading agents in sunscreens.

WO91/09586 describes how certain polymethylalkylsiloxanes can provide hair with improved shine and gloss as well as superior manageability and a soft feel in the same manner as polydimethylsiloxanes, but which also leave the hair feeling less coated and greasy in appearance. These polymethylalkylsiloxanes are also claimed to leave the hair with more body, fullness and softness relative to polydimethylsiloxane fluids.

The present inventors have observed that alkyl modified silicones of the type disclosed in WO91/09586 have a tendency to deposit excessively onto the hair when used in a conventional hair conditioner base. Excessive deposition is correlated with reduced hair volume, which is perceived by many consumers as undesirable.

The present inventors have found that these problems can be solved by combining emulsified particles of the alkyl modified silicone with emulsified particles of certain non-alkyl modified silicones.

### SUMMARY OF THE INVENTION

The present invention provides a hair care composition comprising:
(i) emulsified particles of an alkyl modified silicone, and
(ii) emulsified particles of a non-volatile, non-alkyl modified silicone as defined in claim 1.

Compositions according to the invention deliver effective conditioning to the hair, without unacceptably high deposition.

### DETAILED DESCRIPTION

### Alkyl modified silicone

The hair care composition of the invention comprises emulsified particles of an alkyl modified silicone.

By "alkyl modified silicone" is generally meant an organosiloxane polymer in which at least one pendant alkyl group having a hydrocarbyl chain length of C₆ or greater extends from at least one of the silicon atoms forming the polymer backbone.

The physical form of alkyl modified silicones under ambient conditions generally varies from wax to fluid depending on molecular parameters such as the chain length of the alkyl group, number of alkyl groups (other than methyl) in the molecule and silicone backbone molecular weight.

The term "ambient conditions" as used herein refers to surrounding conditions at one atmosphere of pressure, 50% relative humidity, and 25°C.

Alkyl modified silicones for use in the invention are fluids under ambient conditions.

Preferred alkyl modified silicones for use in the invention have a number average molecular weight (Mₙ) ranging from 10,000 to 450,000, more preferably from 60,000 to 110,000 dalton.

Alkyl modified silicones for use in the hair care compositions of the invention are chemically
characterised by the general formula (I):

(CH₃)₃Si-O-[Si(CH₃)(R)O]ₘ-[Si(CH₃)₂O]ₙ-Si(CH₃)₃ (I)

in which m has a value of 1 to 450, n has a value of 1 to 3000 and R is a monovalent alkyl radical of from 8 to 60 carbon atoms.

In general formula (I), the -[Si(CH₃)₂O]- units are typically randomly interspersed with the -[Si(CH₃)(R)O]- units. m and n are typically average values due to the nature of the polymerisation process.

Preferred materials of general formula (I) for use in the invention have an m value ranging from 40 to 100, more preferably from 50 to 80.

Preferred materials of general formula (I) for use in the invention have an n value ranging from 500 to 1400, more preferably from 700 to 1200.

Preferred materials of general formula (I) for use in the invention have a ratio of m:n ranging from 15:85 to 1:99, more preferably from 10:90 to 5:95.

In preferred materials of general formula (I) for use in the invention, R is a linear alkyl radical having from 8 to 22, more preferably from 8 to 14, most preferably from 10 to 12 carbon atoms.

Methods for the preparation of alkyl modified silicones suitable for use in the invention are known in the art and described for example in EP 495 596 and WO91/09586.

Alkyl modified silicones suitable for use in the invention are also commercially available from suppliers of silicones such as Momentive Performance Materials, Inc. (of Wilton, Connecticut, USA) and Dow Corning Corporation (of Midland, Michigan, USA).

The alkyl modified silicone is present as emulsified particles in the hair care composition of the invention.

The emulsified particles of alkyl modified silicone may typically have a Sauter mean particle diameter (D_{3,2}) in the composition of the invention ranging from 0.1 to 10, preferably from 1 to 4 micrometers.

A suitable method for measuring the Sauter mean droplet diameter (D_{3,2}) is by laser light scattering using an instrument such as a Malvern Mastersizer.

Mixtures of any of the above described alkyl modified silicones may also be used.

Alkyl modified silicones for use in compositions of the invention are available as pre-formed silicone emulsions from suppliers of silicones such as those mentioned above. The use of such pre-formed silicone emulsions is preferred for ease of processing and control of silicone particle size. Such pre-formed silicone emulsions will typically additionally comprise a suitable emulsifier, such as an anionic or non-ionic surfactant, and may be prepared by a chemical emulsification process such as emulsion polymerisation, or by mechanical emulsification using a high shear mixer.

The total amount of alkyl modified silicone in hair care compositions of the invention generally ranges from 0.01 to 5%, preferably from 0.05 to 2%, more preferably from 0.1 to 1.5% by total weight alkyl modified silicone based on the total weight of the composition.

### Non-volatile, non-alkyl modified silicone

The hair care composition of the invention comprises emulsified particles of a non-volatile, non-alkyl modified silicone.

The term "non-volatile" as used herein means that the material in question has a vapour pressure under ambient conditions of 0.2mm Hg or less, preferably about 0.1mm Hg or less.

By "non-alkyl modified silicone" is generally meant an organosiloxane polymer which does not contain any pendant alkyl group having a hydrocarbyl chain length of C₆ or greater extending from at least one of the silicon atoms forming the polymer backbone.

Suitable non-volatile, non-alkyl modified silicones for use in the invention have a viscosity ranging from 350 to 200,000,000 mm²sec⁻¹ at 25°C. Preferably the viscosity is at least 5,000, more preferably at least 10,000 mm²sec⁻¹ at 25°C. Preferably the viscosity does not exceed 20,000,000, more preferably 10,000,000, most preferably 5,000,000 mm²sec⁻¹ at 25°C.

All silicone viscosities mentioned herein are kinematic viscosities unless otherwise specified, and are generally provided by suppliers of silicones, either as measured at 25°C using calibrated capillary glass viscometers under gravity flow conditions, or as deduced from the molecular weight of the material in question.

Preferred non-volatile, non-alkyl modified silicones for use in the invention have a number average molecular weight (Mₙ) ranging from 10,000 to 1,000,000, more preferably from 100,000 to 500,000 dalton.

Suitable non-volatile, non-alkyl modified silicones for use in the hair care compositions of the invention may be chemically characterised by the general formula (II):

A(R)₂Si-O-[Si(R)₂-O]ₓ-Si(R)₂A (II)

in which each R is independently selected from C₁₋₄ alkyl or aryl, x is an integer from 200 to 8,000 and each A is independently selected from C₁₋₄ alkyl, C₁₋₄ alkoxy, aryl, aryloxy or hydroxyl.

In preferred materials of general formula (II) for use in the invention, all R groups are methyl and both A groups are either methyl or hydroxyl. Such materials have the CTFA designation "dimethicone" and "dimethiconol" respectively. Most preferably, all R groups are methyl and both A groups are hydroxyl.

Also suitable as non-volatile, non-alkyl modified silicones for use in the hair care compositions of the invention are aminofunctional polydimethylsiloxanes having the CTFA designation "amodimethicone", and the general formula (III):

HO-[Si(CH₃)₂-O-]ₓ-[Si(R)(R¹-NH-R²NH₂)-O-]_{y}-H (III)

in which R is CH₃ or OH, x and y are independent integers of 1 or more and R¹ and R² are each independently an alkylene group having from 2 to 5 carbon atoms.

Also suitable as non-volatile, non-alkyl modified silicones for use in the hair care compositions of the invention are aminofunctional polydimethylsiloxanes having the CTFA designation "trimethylsilylamodimethicone", and the general formula (IV):

(CH₃)₃Si-O-[Si(CH₃)₂-O-]ₓ-[Si(CH₃)(R¹-NH-R²NH₂)-O-]_{y}-Si(CH₃)₃ (IV)

in which x and y are independent integers of 1 or more and R¹ and R² are each independently an alkylene group having from 2 to 5 carbon atoms.

Mixtures of any of the above described non-volatile, non-alkyl modified silicones may also be used.

The non-volatile, non-alkyl modified silicone is present as emulsified particles in the hair care composition of the invention.

The emulsified particles of non-volatile, non-alkyl modified silicone may typically have a Sauter mean particle diameter (D_{3,2}) in the composition of the invention ranging from 0.01 to 10, preferably from 0.1 to 5, more preferably from 0.5 to 2.5 micrometres.

Non-volatile, non-alkyl modified silicones for use in compositions of the invention are available as pre-formed silicone emulsions from suppliers of silicones such as those mentioned above. The use of such pre-formed silicone emulsions is preferred for ease of processing and control of silicone particle size. Such pre-formed silicone emulsions will typically additionally comprise a suitable emulsifier, such as an anionic or non-ionic surfactant, and may be prepared by a chemical emulsification process such as emulsion polymerisation, or by mechanical emulsification using a high shear mixer.

Examples of suitable commercially available pre-formed emulsions are Dow Corning® 1784 Emulsion and Dow Corning® 1785 Emulsion. These are both anionic emulsions of dimethiconol.

The total amount of non-volatile, non-alkyl modified silicone in hair care compositions of the invention generally ranges from 0.1 to 10%, preferably from 0.5 to 5%, more preferably from 1 to 3% by total weight non-volatile, non-alkyl modified silicone based on the total weight of the composition.

In the composition of the invention, the weight ratio of alkyl modified silicone (as defined above) to non-volatile, non-alkyl modified silicone (as defined above) generally ranges from 10:1 to 1:10, preferably from 1:1 to 1:10, more preferably from 1:2 to 1:8.

### Product Form

Compositions of the invention are typically "rinse-off" compositions to be applied to the hair and then rinsed away.

A particularly preferred product form for compositions in accordance with the invention is a conditioner for the treatment of hair (typically after shampooing) and subsequent rinsing.

### Conditioner Compositions

Conditioners according to the invention will typically comprise one or more cationic surfactants which are cosmetically acceptable and suitable for topical application to the hair.

Suitable cationic surfactants have the formula [N(R¹)(R²)(R³)(R⁴)]⁺(X)⁻ in which R¹, R², R³ and R⁴ are independently (C₁ to C₃₀) alkyl or benzyl and X is a salt-forming anion selected from halogen, acetate, citrate, lactate, glycolate, phosphate, nitrate, sulphate, and methosulphate radicals. Preferably, one, two or three of R¹, R², R³ and R⁴ are independently (C₄ to C₃₀) alkyl and the other R¹, R², R³ and R⁴ group or groups are (C₁-C₆) alkyl or benzyl. More preferably, one or two of R¹, R², R³ and R⁴ are independently (C₆ to C₃₀) alkyl and the other R¹, R², R³ and R⁴ groups are (C₁-C₆) alkyl or benzyl groups. Optionally, the alkyl groups may comprise one or more ester (-OCO- or -COO-) and/or ether (-O-) linkages within the alkyl chain. Alkyl groups may optionally be substituted with one or more hydroxyl groups. Alkyl groups may be straight chain or branched and, for alkyl groups having 3 or more carbon atoms, cyclic. The alkyl groups may be saturated or may contain one or more carbon-carbon double bonds (e.g., oleyl). Alkyl groups are optionally ethoxylated on the alkyl chain with one or more ethyleneoxy groups.

Examples of suitable cationic surfactants for use in conditioner compositions according to the invention include cetyltrimethylammonium chloride, behenyltrimethylammonium chloride, cetylpyridinium chloride, tetramethylammonium chloride, tetraethylammonium chloride, octyltrimethylammonium chloride, dodecyltrimethylammonium chloride, hexadecyltrimethylammonium chloride, octyldimethylbenzylammonium chloride, decyldimethylbenzylammonium chloride, stearyldimethylbenzylammonium chloride, didodecyldimethylammonium chloride, dioctadecyldimethylammonium chloride, tallowtrimethylammonium chloride, dihydrogenated tallow dimethyl ammonium chloride (e.g., Arquad 2HT/75 from Akzo Nobel), cocotrimethylammonium chloride, PEG-2-oleammonium chloride and the corresponding hydroxides thereof. Other suitable cationic surfactants include those materials having the CTFA designations Quaternium-5, Quaternium-31 and Quaternium-18.

A particularly useful cationic surfactant for use in conditioners according to the invention is cetyltrimethylammonium chloride, available commercially, for example as GENAMIN CTAC, ex Hoechst Celanese. Another particularly useful cationic surfactant for use in conditioners according to the invention is behenyltrimethylammonium chloride, available commercially, for example as GENAMIN KDMP, ex Clariant.

Another example of a class of suitable cationic surfactants for use in the invention is a combination of (i) and (ii) below:
(i) an amidoamine corresponding to the general formula (I):

   R¹CONH(CH₂)ₘN(R²)(R³) (I)

   in which R¹ is a hydrocarbyl chain having 10 or more carbon atoms,
   R² and R³ are independently selected from hydrocarbyl chains of from 1 to 10 carbon atoms, and
   m is an integer from 1 to about 10; and
(ii) an acid.

As used herein, the term hydrocarbyl chain means an alkyl or alkenyl chain.

Preferred amidoamine compounds are those corresponding to formula (I) in which
R¹ is a hydrocarbyl residue having from about 11 to about 24 carbon atoms,
R² and R³ are each independently hydrocarbyl residues, preferably alkyl groups, having from 1 to about 4 carbon atoms, and
m is an integer from 1 to about 4.

Preferably, R² and R³ are methyl or ethyl groups.

Preferably, m is 2 or 3, i.e. an ethylene or propylene group.

Preferred amidoamines useful herein include stearamidopropyldimethylamine, stearamidopropyldiethylamine, stearamidoethyldiethylamine, stearamidoethyldimethylamine, palmitamidopropyldimethylamine, palmitamidopropyldiethylamine, palmitamidoethyldiethylamine, palmitamidoethyldimethylamine, behenamidopropyldimethylamine, behenamidopropyldiethylmine, behenamidoethyldiethylamine, behenamidoethyldimethylamine, arachidamidopropyldimethylamine, arachidamidopropyldiethylamine, arachidamidoethyldiethylamine, arachidamidoethyldimethylamine, and mixtures thereof.

Particularly preferred amidoamines useful herein are stearamidopropyldimethylamine, stearamidoethyldiethylamine, and mixtures thereof.

Commercially available amidoamines useful herein include: stearamidopropyldimethylamine with tradenames LEXAMINE S-13 available from Inolex (Philadelphia Pennsylvania, USA) and AMIDOAMINE MSP available from Nikko (Tokyo, Japan), stearamidoethyldiethylamine with a tradename AMIDOAMINE S available from Nikko, behenamidopropyldimethylamine with a tradename INCROMINE BB available from Croda (North Humberside, England), and various amidoamines with the tradename SCHERCODINE available from Scher (Clifton New Jersey, USA).

Acid (ii) may be any organic or mineral acid which is capable of protonating the amidoamine in the hair treatment composition. Suitable acids useful herein include hydrochloric acid, acetic acid, tartaric acid, fumaric acid, lactic acid, malic acid, succinic acid, and mixtures thereof. Preferably, the acid is selected from the group consisting of acetic acid, tartaric acid, hydrochloric acid, fumaric acid, and mixtures thereof.

The primary role of the acid is to protonate the amidoamine in the hair treatment composition thus forming a tertiary amine salt (TAS) in situ in the hair treatment composition. The TAS in effect is a non-permanent quaternary ammonium or pseudo-quaternary ammonium cationic surfactant.

Suitably, the acid is included in a sufficient amount to protonate all the amidoamine present, i.e. at a level which is at least equimolar to the amount of amidoamine present in the composition.

Mixtures of any of the above described cationic surfactants may also be used.

In conditioners of the invention, the level of cationic surfactant will generally range from 0.01 to 10%, more preferably 0.05 to 7.5%, most preferably 0.1 to 5% by total weight cationic surfactant based on the total weight of the composition.

Conditioners of the invention will typically also incorporate a fatty alcohol. The combined use of fatty alcohols and cationic surfactants in conditioning compositions is believed to be especially advantageous, because this leads to the formation of a lamellar phase, in which the cationic surfactant is dispersed.

Representative fatty alcohols comprise from 8 to 22 carbon atoms, more preferably 16 to 22. Fatty alcohols are typically compounds containing straight chain alkyl groups. Examples of suitable fatty alcohols include cetyl alcohol, stearyl alcohol and mixtures thereof. The use of these materials is also advantageous in that they contribute to the overall conditioning properties of compositions of the invention.

The level of fatty alcohol in conditioners of the invention will generally range from 0.01 to 10%, preferably from 0.1 to 8%, more preferably from 0.2 to 7%, most preferably from 0.3 to 6% by weight of the composition. The weight ratio of cationic surfactant to fatty alcohol is suitably from 1:1 to 1:10, preferably from 1:1.5 to 1:8, optimally from 1:2 to 1:5. If the weight ratio of cationic surfactant to fatty alcohol is too high, this can lead to eye irritancy from the composition. If it is too low, it can make the hair feel squeaky for some consumers.

Another preferred product form is a shampoo composition.

### Shampoo Composition

Shampoo compositions of the invention are generally aqueous, i.e. they have water or an aqueous solution or a lyotropic liquid crystalline phase as their major component.
Suitably, the composition will comprise from 50 to 98%, preferably from 60 to 90% water by weight based on the total weight of the composition.

### Anionic Cleansing Surfactant

Shampoo compositions according to the invention will generally comprise one or more anionic cleansing surfactants which are cosmetically acceptable and suitable for topical application to the hair.

Examples of suitable anionic cleansing surfactants are the alkyl sulphates, alkyl ether sulphates, alkaryl sulphonates, alkanoyl isethionates, alkyl succinates, alkyl sulphosuccinates, alkyl ether sulphosuccinates, N-alkyl sarcosinates, alkyl phosphates, alkyl ether phosphates, and alkyl ether carboxylic acids and salts thereof, especially their sodium, magnesium, ammonium and mono-, di- and triethanolamine salts. The alkyl and acyl groups generally contain from 8 to 18, preferably from 10 to 16 carbon atoms and may be unsaturated. The alkyl ether sulphates, alkyl ether sulphosuccinates, alkyl ether phosphates and alkyl ether carboxylic acids and salts thereof may contain from 1 to 20 ethylene oxide or propylene oxide units per molecule.

Typical anionic cleansing surfactants for use in shampoo compositions of the invention include sodium oleyl succinate, ammonium lauryl sulphosuccinate, sodium lauryl sulphate, sodium lauryl ether sulphate, sodium lauryl ether sulphosuccinate, ammonium lauryl sulphate, ammonium lauryl ether sulphate, sodium dodecylbenzene sulphonate, triethanolamine dodecylbenzene sulphonate, sodium cocoyl isethionate, sodium lauryl isethionate, lauryl ether carboxylic acid and sodium N-lauryl sarcosinate.

Preferred anionic cleansing surfactants are sodium lauryl sulphate, sodium lauryl ether sulphate (n)EO, (where n is from 1 to 3), sodium lauryl ether sulphosuccinate(n)EO, (where n is from 1 to 3), ammonium lauryl sulphate, ammonium lauryl ether sulphate(n)EO, (where n is from 1 to 3), sodium cocoyl isethionate and lauryl ether carboxylic acid (n) EO (where n is from 10 to 20).

Mixtures of any of the foregoing anionic cleansing surfactants may also be suitable.

The total amount of anionic cleansing surfactant in shampoo compositions of the invention generally ranges from 0.5 to 45%, preferably from 1.5 to 35%, more preferably from 5 to 20% by total weight anionic cleansing surfactant based on the total weight of the composition.

### Further Ingredients

Optionally, a shampoo composition of the invention may contain further ingredients as described below to enhance performance and/or consumer acceptability.

### Co-surfactant

The composition can include co-surfactants, to help impart aesthetic, physical or cleansing properties to the composition.

An example of a co-surfactant is a nonionic surfactant, which can be included in an amount ranging from 0.5 to 8%, preferably from 2 to 5% by weight based on the total weight of the composition.

For example, representative nonionic surfactants that can be included in shampoo compositions of the invention include condensation products of aliphatic (C₈ - C₁₈) primary or secondary linear or branched chain alcohols or phenols with alkylene oxides, usually ethylene oxide and generally having from 6 to 30 ethylene oxide groups.

Other representative nonionic surfactants include mono- or di-alkyl alkanolamides. Examples include coco mono- or diethanolamide and coco mono-isopropanolamide.

Further nonionic surfactants which can be included in shampoo compositions of the invention are the alkyl polyglycosides (APGs). Typically, the APG is one which comprises an alkyl group connected (optionally via a bridging group) to a block of one or more glycosyl groups. Preferred APGs are defined by the following formula:

RO - (G)ₙ

wherein R is a branched or straight chain alkyl group which may be saturated or unsaturated and G is a saccharide group.

R may represent a mean alkyl chain length of from about C₅ to about C₂₀. Preferably R represents a mean alkyl chain length of from about C₈ to about C₁₂. Most preferably the value of R lies between about 9.5 and about 10.5. G may be selected from C₅ or C₆ monosaccharide residues, and is preferably a glucoside. G may be selected from the group comprising glucose, xylose, lactose, fructose, mannose and derivatives thereof. Preferably G is glucose.

The degree of polymerisation, n, may have a value of from about 1 to about 10 or more. Preferably, the value of n lies from about 1.1 to about 2. Most preferably the value of n lies from about 1.3 to about 1.5.

Suitable alkyl polyglycosides for use in the invention are commercially available and include for example those materials identified as: Oramix NS10 ex Seppic; Plantaren 1200 and Plantaren 2000 ex Henkel.

Other sugar-derived nonionic surfactants which can be included in compositions of the invention include the C₁₀-C₁₈ N-alkyl (C₁-C₆) polyhydroxy fatty acid amides, such as the C₁₂-C₁₈ N-methyl glucamides, as described for example in WO 92 06154 and US 5 194 639, and the N-alkoxy polyhydroxy fatty acid amides, such as C₁₀-C₁₈ N-(3-methoxypropyl) glucamide.

A preferred example of a co-surfactant is an amphoteric or zwitterionic surfactant, which can be included in an amount ranging from 0.5 to about 8%, preferably from 1 to 4% by weight based on the total weight of the composition.

Examples of amphoteric or zwitterionic surfactants include alkyl amine oxides, alkyl betaines, alkyl amidopropyl betaines, alkyl sulphobetaines (sultaines), alkyl glycinates, alkyl carboxyglycinates, alkyl amphoacetates, alkyl amphopropionates, alkylamphoglycinates, alkyl amidopropyl hydroxysultaines, acyl taurates and acyl glutamates, wherein the alkyl and acyl groups have from 8 to 19 carbon atoms. Typical amphoteric and zwitterionic surfactants for use in shampoos of the invention include lauryl amine oxide, cocodimethyl sulphopropyl betaine, lauryl betaine, cocamidopropyl betaine and sodium cocoamphoacetate.

A particularly preferred amphoteric or zwitterionic surfactant is cocamidopropyl betaine.

Mixtures of any of the foregoing amphoteric or zwitterionic surfactants may also be suitable. Preferred mixtures are those of cocamidopropyl betaine with further amphoteric or zwitterionic surfactants as described above. A preferred further amphoteric or zwitterionic surfactant is sodium cocoamphoacetate.

The total amount of surfactant (including any co-surfactant, and/or any emulsifier) in a shampoo composition of the invention is generally from 1 to 50%, preferably from 2 to 40%, more preferably from 10 to 25% by total weight surfactant based on the total weight of the composition.

### Cationic Polymers

Cationic polymers are preferred ingredients in a shampoo composition of the invention for enhancing conditioning performance.

Suitable cationic polymers may be homopolymers which are cationically substituted or may be formed from two or more types of monomers. The weight average (M_{w}) molecular weight of the polymers will generally be between 100 000 and 2 million daltons. The polymers will have cationic nitrogen containing groups such as quaternary ammonium or protonated amino groups, or a mixture thereof. If the molecular weight of the polymer is too low, then the conditioning effect is poor. If too high, then there may be problems of high extensional viscosity leading to stringiness of the composition when it is poured.

The cationic nitrogen-containing group will generally be present as a substituent on a fraction of the total monomer units of the cationic polymer. Thus when the polymer is not a homopolymer it can contain spacer non-cationic monomer units. Such polymers are described in the CTFA Cosmetic Ingredient Directory, 3rd edition. The ratio of the cationic to non-cationic monomer units is selected to give polymers having a cationic charge density in the required range, which is generally from 0.2 to 3.0 meq/gm. The cationic charge density of the polymer is suitably determined via the Kjeldahl method as described in the US Pharmacopoeia under chemical tests for nitrogen determination.

Suitable cationic polymers include, for example, copolymers of vinyl monomers having cationic amine or quaternary ammonium functionalities with water soluble spacer monomers such as (meth)acrylamide, alkyl and dialkyl (meth)acrylamides, alkyl (meth)acrylate, vinyl caprolactone and vinyl pyrrolidine. The alkyl and dialkyl substituted monomers preferably have C1-C7 alkyl groups, more preferably C1-3 alkyl groups. Other suitable spacers include vinyl esters, vinyl alcohol, maleic anhydride, propylene glycol and ethylene glycol.

The cationic amines can be primary, secondary or tertiary amines, depending upon the particular species and the pH of the composition. In general secondary and tertiary amines, especially tertiary, are preferred.

Amine substituted vinyl monomers and amines can be polymerised in the amine form and then converted to ammonium by quaternization.

The cationic polymers can comprise mixtures of monomer units derived from amine- and/or quaternary ammonium-substituted monomer and/or compatible spacer monomers.

Suitable cationic polymers include, for example:
- cationic diallyl quaternary ammonium-containing polymers including, for example, dimethyldiallylammonium chloride homopolymer and copolymers of acrylamide and dimethyldiallylammonium chloride, referred to in the industry (CTFA) as Polyquaternium 6 and Polyquaternium 7, respectively;
- mineral acid salts of amino-alkyl esters of homo-and co-polymers of unsaturated carboxylic acids having from 3 to 5 carbon atoms, (as described in U.S. Patent 4,009,256);
- cationic polyacrylamides(as described in WO95/22311).

Other cationic polymers that can be used include cationic polysaccharide polymers, such as cationic cellulose derivatives, cationic starch derivatives, and cationic guar gum derivatives.

Cationic polysaccharide polymers suitable for use in compositions of the invention include monomers of the formula:

A-O-[R-N⁺(R¹)(R²)(R³)X⁻],

wherein: A is an anhydroglucose residual group, such as a starch or cellulose anhydroglucose residual. R is an alkylene, oxyalkylene, polyoxyalkylene, or hydroxyalkylene group, or combination thereof. R¹, R² and R³ independently represent alkyl, aryl, alkylaryl, arylalkyl, alkoxyalkyl, or alkoxyaryl groups, each group containing up to about 18 carbon atoms. The total number of carbon atoms for each cationic moiety (i.e., the sum of carbon atoms in R¹, R² and R³) is preferably about 20 or less, and X is an anionic counterion.

Another type of cationic cellulose includes the polymeric quaternary ammonium salts of hydroxyethyl cellulose reacted with lauryl dimethyl ammonium-substituted epoxide, referred to in the industry (CTFA) as Polyquaternium 24. These materials are available from the Amerchol Corporation, for instance under the tradename Polymer LM-200.

Other suitable cationic polysaccharide polymers include quaternary nitrogen-containing cellulose ethers (e.g. as described in U.S. Patent 3,962,418), and copolymers of etherified cellulose and starch (e.g. as described in U.S. Patent 3,958,581).

A particularly suitable type of cationic polysaccharide polymer that can be used is a cationic guar gum derivative, such as guar hydroxypropyltrimethylammonium chloride (commercially available from Rhodia in their JAGUAR trademark series). Examples of such materials are JAGUAR C13S, JAGUAR C14, JAGUAR C15, JAGUAR C17, JAGUAR C16, JAGUAR CHT and JAGUAR C162.

Mixtures of any of the above cationic polymers may be used.

Cationic polymer will generally be present in a shampoo composition of the invention at levels of from 0.01 to 5%, preferably from 0.05 to 1%, more preferably from 0.08 to 0.5% by total weight of cationic polymer based on the total weight of the composition.

### Suspending Agent

Preferably an aqueous shampoo composition of the invention further comprises a suspending agent. Suitable suspending agents are selected from polyacrylic acids, cross-linked polymers of acrylic acid, copolymers of acrylic acid with a hydrophobic monomer, copolymers of carboxylic acid-containing monomers and acrylic esters, cross-linked copolymers of acrylic acid and acrylate esters, heteropolysaccharide gums and crystalline long chain acyl derivatives. The long chain acyl derivative is desirably selected from ethylene glycol stearate, alkanolamides of fatty acids having from 16 to 22 carbon atoms and mixtures thereof. Ethylene glycol distearate and polyethylene glycol 3 distearate are preferred long chain acyl derivatives, since these impart pearlescence to the composition. Polyacrylic acid is available commercially as Carbopol 420, Carbopol 488 or Carbopol 493. Polymers of acrylic acid cross-linked with a polyfunctional agent may also be used; they are available commercially as Carbopol 910, Carbopol 934, Carbopol 941 and Carbopol 980. An example of a suitable copolymer of a carboxylic acid containing monomer and acrylic acid esters is Carbopol 1342. All Carbopol (trademark) materials are available from Goodrich.

Suitable cross-linked polymers of acrylic acid and acrylate esters are Pemulen TR1 or Pemulen TR2. A suitable heteropolysaccharide gum is xanthan gum, for example that available as Kelzan mu.

Mixtures of any of the above suspending agents may be used. Preferred is a mixture of cross-linked polymer of acrylic acid and crystalline long chain acyl derivative.

Suspending agent will generally be present in a shampoo composition of the invention at levels of from 0.1 to 10%, preferably from 0.5 to 6%, more preferably from 0.9 to 4% by total weight of suspending agent based on the total weight of the composition.

### Further Conditioning Agents

Compositions of the invention may comprise further conditioning agents, in addition to the materials described above, in order to optimise wet and dry conditioning benefits.

Examples of suitable further conditioning agents are volatile silicones.

The term "volatile" as used herein means that the material in question has a vapour pressure under ambient conditions equal to or above that of ethanol.

Volatile silicones suitable for use in the composition of the invention include both linear and cyclic silicones. The linear volatile silicones will generally have viscosities of 5 mm²sec⁻¹ (centistokes) or less at 25°C, while the cyclic volatile silicones will generally have viscosities of 10 mm²sec⁻¹ or less at 25°C.

Suitable cyclic volatile silicones include cyclopolysiloxanes such as cycloalkylsiloxanes and cycloalkylalkoxysiloxanes, wherein alkyl and alkoxy groups contain C₁-C₈ alkyl groups.

A general formula for suitable cyclic volatile silicones is:

[(R₁)(R₂)Si-O-]ₙ

in which n is 3 to 7 and R₁ and R₂ are independently selected from C₁-C₈ alkyl, aryl (especially phenyl), and alkaryl (e.g. C₁-C₈ substituted aryl).

Preferably R₁ and R₂ are C₁-C₂ alkyl. Most preferably R₁ and R₂ are C₁ alkyl(such materials have the CTFA designation cyclomethicone).

Preferably n is 4 to 6, most preferably 4 or 5.

Specific examples of most preferred cyclic volatile silicones are octamethyl cyclotetrasiloxane, decamethyl cyclopentasiloxane, and mixtures thereof.

Linear volatile silicones include polyorganosiloxanes such as polydialkylsiloxanes and polyalkylarylsiloxanes.

A general formula for suitable linear volatile silicones is:

(R₁)(R₂)(R₃)Si-O-[Si(R₄)(R₅)O]ₙSi(R₆)(R₇)(R₈)

in which n is 1 to 7 and R₁, R₂, R₃, R₄, R₅, R₆, R₇, and R₈ are independently selected from C₁-C₈ alkyl, aryl (especially phenyl), and alkaryl (e.g. C₁-C₈ substituted aryl).

Preferably R₁, R₂, R₃, R₄, R₅, R₆, R₇, and R₈ are C₁-C₂ alkyl. Most preferably R₁, R₂, R₃, R₄, R₅, R₆, R₇, and R₈ are C₁ alkyl (such materials have the CTFA designation dimethicone).

Silicones of the above described types are commercially available, for example from Dow Corning Corporation as DC 244, 245, 344, 345 and 200 fluids.

### Other Optional Ingredients

A composition of the invention may contain other ingredients for enhancing performance and/or consumer acceptability. Such ingredients include fragrance, dyes and pigments, pH adjusting agents, pearlescers or opacifiers, viscosity modifiers, preservatives, and natural hair nutrients such as botanicals, fruit extracts, sugar derivatives and amino acids.

### Mode of Use

The compositions of the invention are primarily intended for topical application to the hair and/or scalp of a human subject in rinse-off compositions, in order to improve hair fibre surface properties such as hair fibre lubrication, smoothness, softness, manageability, alignment, and shine.

The compositions provided by the invention are preferably conditioner compositions for the treatment of hair (typically after shampooing) and subsequent rinsing.

Alternatively the compositions provided by the invention may be aqueous shampoo compositions, used by massaging them into the hair followed by rinsing with clean water prior to drying the hair. Optionally, a separate conditioning formulation may be applied after rinsing and before drying, but this may not be necessary as an aqueous shampoo composition of this invention is intended to provide both cleansing and conditioning to the hair.

The invention is further illustrated with reference to the following, non-limiting examples, in which all percentages are by weight based on total weight unless otherwise specified. Examples according to the invention are indicated by a number. Comparative examples (not according to the invention) are indicated by a letter.

### EXAMPLES

Hair conditioner formulations were prepared having ingredients as shown in the following Table 1:

**Table 1**

| Ingredient | Ex.1 | Ex.2 | Ex.A | Ex.B | Ex.C | Ex.D | Ex.E | Ex.F | Ex.G |
|---|---|---|---|---|---|---|---|---|---|
| ARQUAD® 16-50⁽¹⁾ | 3.20 | 3.20 | 3.20 | 3.20 | 3.20 | 3.20 | 3.20 | 3.20 | 3.20 |
| ARQUAD® 2HT⁽²⁾ | 1.07 | 1.07 | 1.07 | 1.07 | 1.07 | 1.07 | 1.07 | 1.07 | 1.07 |
| Cetostearyl alcohol | 6.00 | 6.00 | 6.00 | 6.00 | 6.00 | 6.00 | 6.00 | 6.00 | 6.00 |
| Alkyl-modified silicone⁽³⁾ | 0.77 | -- | 1.54 | 4.62 | 7.69 | -- | -- | -- | -- |
| Alkyl-modified silicone⁽⁴⁾ | -- | 0.77 | -- | -- | -- | 1.54 | 4.62 | 7.69 | -- |
| DOW CORNING® 1785 Emulsion⁽⁵⁾ | 4.17 | 4.17 | -- | -- | -- | -- | -- | -- | 5 |
| Water, minors | q.s. | q.s. | q.s. | q.s. | q.s. | q.s. | q.s. | q.s. | q.s. |

| | | | | | | | | | |
|---|---|---|---|---|---|---|---|---|---|
| ⁽¹⁾ Cetrimonium chloride (50wt% a.i.). ⁽²⁾ Di(hydrogenated tallow) dimethylammonium chloride (75wt% a.i.). ⁽³⁾ Emulsion (65wt% a.i., with non-ionic surfactant) of an alkyl modified silicone of formula (I) above in which R is a C₁₂ linear alkyl radical, m is 75 and n is 1150 (from Momentive Performance Materials, Inc.). ⁽⁴⁾ Emulsion (65wt% a.i., with non-ionic surfactant) of an alkyl modified silicone of formula (I) above in which R is a C₁₂ linear alkyl radical, m is 50 and n is 770 (from Momentive Performance Materials, Inc.). ⁽⁵⁾ Emulsion (60wt% a.i., with anionic surfactant) of dimethiconol. | | | | | | | | | |

The formulations of Table 1 were evaluated for their ability to align hair and for their silicon deposition on hair.
For the hair alignment study, the test formulation is applied to hair switches of a standard mass and dimension of hair. Photographs are generated once these switches have been dried under controlled conditions. Panellists are asked to make paired comparisons of the degree of misalignment of the switches in these photographs against a fixed reference image. 36 comparisons are made for each test formulation.

Silicon deposition was measured by X-ray fluorescence spectroscopy.

The volume of the hair switches after treatment with the test formulations was also assessed.

The results of the above analyses are shown in the following Table 2:

**Table 2**

| Test Measurement | Ex.1 | Ex.2 | Ex.A | Ex.B | Ex.C | Ex.D | Ex.E | Ex.F | Ex.G |
|---|---|---|---|---|---|---|---|---|---|
| Misalignment panel score | 0.993 | 1.075 | 1.316 | 1.009 | 0.977 | 1.278 | 1.322 | 0.943 | 1.382 |
| Standard error of misalignment panel score | 0.054 | 0.031 | 0.046 | 0.06 | 0.046 | 0.06 | 0.046 | 0.06 | 0.045 |
| Silicon deposition (ppm) | 1061 | 271 | 484 | 1740 | 11835 | 577 | 1916 | 6544 | 214 |
| Standard deviation of silicon deposition | 565 | 84 | 87 | 307 | 6947 | 175 | 592 | 972 | 200 |
| Switch volume (mm²) | 8647.57 | 10023.32 | 10888.68 | 6950.78 | 1253.22 | 10501.79 | 7025.24 | 1852.81 | 10256.67 |
| Standard deviation of switch volume | 1238.66 | 659.84 | 694.21 | 1079.5 | 129.66 | 1047.74 | 1439.31 | 525.53 | 977.29 |

For hair misalignment, a lower score is preferred. It is also desirable to avoid excessive levels of silicone deposition on the hair. It can be seen from the above data how high levels of silicon deposition correlate with a reduced switch volume.

Example 1 (according to the invention) and Examples A to C (comparatives) all employ alkyl modified silicone ⁽³⁾ at various active levels. Within this series, Example 1 and Example C provide the best (i.e. lowest) hair misalignment scores. However, Example C demonstrates an undesirable 11-fold increase in silicon deposition on the hair, and significantly reduced switch volume, relative to Example 1.

Example 2 (according to the invention) and Examples D to F (comparatives) all employ alkyl modified silicone ⁽⁴⁾ at various active levels. Within this series, Example 2 and Example F provide the best (i.e. lowest) hair misalignment scores. However, Example F demonstrates an undesirable 24-fold increase in silicon deposition, and significantly reduced switch volume, relative to Example 2.

Comparative Example G gives a level of silicon deposition which is approximately equivalent to that of Example 2. However the performance of Example G on hair misalignment is significantly inferior to that of Example 2.

Therefore the formulations of the invention provide a superior balance of hair alignment, silicon deposition and hair volume relative to the comparative examples.

## Claims

1. A hair care composition comprising:
(i) emulsified particles of an alkyl modified silicone, and
(ii) emulsified particles of a non-volatile, non-alkyl modified silicone, **characterised in that** the alkyl modified silicone is a fluid under ambient conditions and has the general formula (I):
(CH₃)₃Si-O-[Si(CH₃)(R)O]ₘ-[Si(CH₃)₂O]ₙ-Si(CH₃)₃ (I)
in which m has a value of 1 to 450, n has a value of 1 to 3000 and R is a monovalent alkyl radical of from 8 to 60 carbon atoms,
wherein the non-alkyl modified silicone is an organosiloxane polymer which does not contain any pendant alkyl group having a hydrocarbyl chain length of C6 or greater extending from at least one of the silicon atoms forming the polymer backbone.

2. A composition according to claim 1, in which R is a linear alkyl radical having from 10 to 12 carbon atoms.

3. A composition according to any one of claims 1 to 2, in which the non-volatile, non-alkyl modified silicone has the general formula (II):
A(R)₂Si-O-[Si(R)₂-O]ₓ-Si(R)₂A (II)
in which each R is independently selected from C₁₋₄ alkyl or aryl, x is an integer from 200 to 8,000 and each A is independently selected from C₁₋₄ alkyl, C₁₋₄ alkoxy, aryl, aryloxy or hydroxyl.

4. A composition according to claim 3, in which all R groups are methyl and both A groups are hydroxyl.

5. A composition according to any one of claims 1 to 4, in which the weight ratio of alkyl modified silicone to non-volatile, non-alkyl modified silicone ranges from 1:2 to 1:8.

6. A composition according to any one of claims 1 to 5, which is in the form of a conditioner for the treatment of hair (after shampooing) and subsequent rinsing.

## Patentansprüche

1. Haarpflegezusammensetzung, umfassend:
(i) emulgierte Partikel eines Alkyl-modifizierten Silikons und
(ii) emulgierte Partikel eines nicht-flüchtigen, nicht-Alkyl-modifizierten Silikons, **dadurch gekennzeichnet, dass** das Alkyl-modifizierte Silikon unter Umgebungsbedingungen eine Flüssigkeit darstellt und die allgemeine Formel (I):
(CH₃)₃Si-O-[Si(CH₃)(R)O]ₘ-[Si(CH₃)₂O]ₙ-Si(CH₃)₃ (I)
aufweist,
in welcher m einen Wert von 1 bis 450 hat, n einen Wert von 1 bis 3000 hat
und R ein einwertiger Alkyl-Rest mit 8 bis 60 Kohlenstoffatomen ist,
wobei das nicht-Alkyl-modifizierte Silikon ein Organosiloxanpolymer ist, welches nicht irgendeine anhängende Alkylgruppe mit einer Hydroxycarbylkettenlänge von C6 oder mehr enthält, die sich von mindestens einem der das Polymergrundgerüst bildenden Silikonatome erstreckt.

2. Zusammensetzung nach Anspruch 1, worin R einen linearen Alkyl-Rest mit 10 bis 12 Kohlenstoffatomen darstellt.

3. Zusammensetzung nach irgendeinem der Ansprüche 1 bis 2, worin das nichtflüchtige, nicht-Alkyl-modifizierte Silikon die allgemeine Formel (II):
A(R)₂Si-O-[Si(R)₂-O]ₓ-Si(R)₂A (II)
aufweist,
worin jedes R, unabhängig voneinander, unter C₁₋₄-Alkyl oder Aryl ausgewählt ist, x eine ganze Zahl von 200 bis 8.000 ist und jedes A, unabhängig voneinander, unter C₁₋₄-Alkyl, C₁₋₄-Alkoxy, Aryl, Aryloxy oder Hydroxyl ausgewählt ist.

4. Zusammensetzung nach Anspruch 3, wobei alle Gruppen R Methyl sind und beide A-Gruppen Hydroxyl sind.

5. Zusammensetzung nach irgendeinem der Ansprüche 1 bis 4, worin das Gewichtsverhältnis von Alkyl-modifiziertem Silikon zu nicht-flüchtigem, nicht-Alkyl-modifiziertem Silikon zwischen 1:2 und 1:8 liegt.

6. Zusammensetzung nach irgendeinem der Ansprüche 1 bis 5, welche in Form eines Konditionierers für die Behandlung von Haar (nach dem Shampoonieren) und zum nachfolgenden Spülen dient.

## Revendications

1. Composition pour le soin des cheveux comprenant :
(i) des particules émulsionnées d'un silicone modifié par un groupe alkyle, et
(ii) des particules émulsionnées d'un silicone non modifié par un groupe alkyle, non-volatile, **caractérisée en ce que** le silicone modifié par un groupe alkyle est un fluide dans des conditions ambiantes et présente la formule générale (I) :
(CH₃)₃Si-O-[Si(CH₃)(R)O]ₘ-[Si(CH₃)₂O]ₙ-Si(CH₃)₃ (I)
dans laquelle m présente une valeur de 1 à 450, n présente une valeur de 1 à 3 000 et R est un radical alkyle monovalent de 8 à 60 atomes de carbone,
dans laquelle le silicone non modifié par un groupe alkyle est un polymère d'organosiloxane qui ne contient pas de groupe alkyle suspendu présentant une longueur de chaîne hydrocarbyle de C6 ou supérieure s'étendant à partir d'au moins un des atomes de silicium formant le squelette de polymère.

2. Composition selon la revendication 1, dans laquelle R est un radical alkyle linéaire ayant de 10 à 12 atomes de carbone.

3. Composition selon l'une quelconque des revendications 1 à 2, dans laquelle le silicone non modifié par un groupe alkyle, non-volatile, présente la formule générale (II) :
A(R)₂Si-O-[Si(R)₂-O]ₓ-Si(R)₂A (II)
dans laquelle chaque R est indépendamment choisi parmi un groupe alkyle en C₁₋₄ ou aryle, x est un nombre entier de 200 à 8 000 et chaque A est indépendamment choisi parmi un groupe alkyle en C₁₋₄, alcoxy en C₁₋₄, aryle, aryloxy ou hydroxyle.

4. Composition selon la revendication 3, dans laquelle tous les groupes R sont un groupe méthyle et les deux groupes A sont un groupe hydroxyle.

5. Composition selon l'une quelconque des revendications 1 à 4, dans laquelle le rapport massique de silicone modifié par un groupe alkyle au silicone non modifié par un groupe alkyle, non-volatile est de 1:2 à 1:8.

6. Composition selon l'une quelconque des revendications 1 à 5, laquelle est dans la forme d'un agent de conditionnement pour le traitement de cheveux (après shampoing) et le rinçage subséquent.
